# EUROPÄISCHE PATENTSCHRIFT

(11) **EP 0 742 045 B1**
(45) Veröffentlichungstag und Bekanntmachung des Hinweises auf die Patenterteilung: **12.12.2001**
(21) Anmeldenummer: 96106610.7
(22) Anmeldetag: 26.04.1996
(51) Int. Cl.: B01J 23/889, B01J 23/78, B01J 27/185, B01J 27/187, C07C 209/48, C07C 209/52

(54) **Kobaltkatalysatoren**
Cobalt catalysts
Catalyseurs à base de cobalt

(30) Priorität: 09.05.1995 DE 19516845; 11.07.1995 DE 19525187
(43) Veröffentlichungstag der Anmeldung: 13.11.1996
(73) Patentinhaber: BASF AKTIENGESELLSCHAFT, 67056 Ludwigshafen (DE)
(72) Erfinder: Breitscheidel, Boris, Dr., 36043 Fulda (DE); Polanek, Peter, Dr., 69469 Weinheim (DE); Voit, Guido, Dr., 69198 Schriesheim (DE); Witzel, Tom, Dr., 67069 Ludwigshafen (DE); Linden, Gerd, Dr., 69120 Heidelberg (DE); Hesse, Michael. Dr., 67105 Schifferstadt (DE)

(56) Entgegenhaltungen:
- EP-A- 0 322 760
- DE-A- 4 325 847

## Beschreibung

Die vorliegende Erfindung betrifft neue Kobaltkatalysatoren, deren katalytisch aktive Masse aus Kobalt, Phosphor, Mangan und Alkali besteht und die nach der Kalzinierung bei Endtemperaturen von 200 bis 400°C im Wasserstoffstrom reduziert und anschließend durch Behandlung im Luftstrom bei Endtemperaturen von 20 bis 60°C oberflächlich anoxidiert werden.

Aus der EP-A-445 589 sind Hydrierkatalysatoren bekannt, deren katalytisch aktive Masse 20 bis 95 Gew.-% Kobaltoxid, 0,5 bis 60 Gew.-% Oxide der Metalle Mangan, Nickel, Eisen, Chrom, Molybdän, Wolfram oder Phosphor und 0,5 bis 20 Gew.-% Oxide der Alkali- oder Erdalkali-, der seltenen Erdengruppe, Scandium oder Yttrium enthalten.

Aus der DE-A-34 03 377 sind geformte Katalysatormassen bekannt, die metallisches Kobalt- und/oder Nickel, mit einem Gehalt von weniger als 0,1 Gew.-% an Alkali- und/oder Erdalkalioxiden, enthalten und die bei Temperaturen von kleiner oder gleich 500°C durch Reduktion hergestellt wurden. Die geformten Katalysatormassen haben eine Druckhärte von mehr als 300 kp/cm².

Die genannten Katalysatoren haben jedoch den Nachteil, daß sie nicht ausreichend basenstabil sind, um eine lange Lebensdauer in basischem Medium zu gewährleisten.

EP-A-322 760 betrifft ein Verfahren zur Herstellung von Kobaltkatalysatoren durch Ausfällen von Kobaltcarbonat, Filtrieren, Auswaschen, gegebenenfalls Formen und Reduktion mit Wasserstoff bei 200 bis 300°C.

In DE-A-43 25 847 werden kobalthaltige basenstabile Katalysatoren beschrieben, deren katalytisch aktive Masse aus 55 bis 98 Gew.-% Kobalt, 0,2 bis 15 Gew.-% Phosphor, 0,2 bis 15 Gew.-% Mangan und 0,2 bis 15 Gew.-% Alkali bestehen und die durch zwei Kalzinierungsschritte bei Endtemperaturen von 550 bis 750°C und 800 bis 1000°C hergestellt werden. Diese Kobaltkatalysatoren weisen nach Durchführung eines Kochtests zur Bestimmung der Basenstabilität eine Schneidhärte von größer oder gleich 10 N auf und besitzen erfahrungsgemäß bei Reaktionen in basischen Medien eine Standzeit von > 3000 h, maximal jedoch 5000 h.

Für die wirtschaftliche Durchführung von Reaktionen in basischen Medien, z.B. die Hydrierung von organischen Nitrilen und/oder Iminen, sind jedoch Katalysator-Standzeiten von > 5000 h notwendig.

Der vorliegenden Erfindung lag daher die Aufgabe zugrunde, den zuvor genannten Nachteilen abzuhelfen, insbesondere kobalthaltige Katalysatoren zu entwickeln, die eine so hohe Basenstabilität besitzen, daß bei Reaktionen in basischen Medien Standzeiten von > 5000 h erreicht werden.

Demgemäß wurden neue und verbesserte Kobaltkatalysatoren, deren katalytisch aktive Masse aus 55 bis 98 Gew.-% Kobalt, 0,2 bis 15 Gew.-% Phosphor, 0,2 bis 15 Gew.-% Mangan und 0,05 bis 5 Gew.-% Alkali, berechnet als Oxid, besteht, gefunden, welche dadurch gekennzeichnet sind, daß man die kalzinierten Katalysatoren bei Endtemperaturen von 200 bis 400°C im Wasserstoffstrom reduziert und anschließend durch Behandlung im Luftstrom bei Endtemperaturen von 20 bis 60°C oberflächlich anoxidiert, daß die Kobaltkatalysatoren eine spezifische Oberfläche von größer oder gleich 12 m²/g besitzen und daß die Kobaltkatalysatoren nach Durchführung eines Kochtests zur Bestimmung der Basenstabilität eine Schneidhärte von größer oder gleich 30 N aufweisen.

Die katalytisch aktive Masse der erfindungsgemäßen Kobaltkatalysatoren besteht aus 55 bis 98 Gew.-% Kobalt, bevorzugt 75 bis 95 Gew.-% Kobalt, besonders bevorzugt 85 bis 95 Gew.-% Kobalt, 0,2 bis 15 Gew.-% Phosphor, bevorzugt 0,5 bis 10 Gew.-% Phosphor, besonders bevorzugt 1 bis 6 % Phosphor, 0,2 bis 15 Gew.-% Mangan, bevorzugt 2 bis 10 Gew.-% Mangan, besonders bevorzugt 3 bis 8 Gew.-% Mangan und 0,05 bis 5 Gew.-% Alkali, bevorzugt 0,1 bis 3 Gew.-% Alkali, besonders bevorzugt 0,13 bis 1 Gew.-% Alkali, berechnet als Oxid.

Als Alkali eignen sich bevorzugt Lithium, Natrium, Kalium und/ oder Cäsium, besonders bevorzugt Natrium und/oder Kalium.

Die erfindungsgemäßen Kobaltkatalysatoren lassen sich wie folgt herstellen:

Eine Lösung eines Kobalt-Salzes, bevorzugt eines anorganischen Kobalt-Salzes und gegebenenfalls der gewünschten Promotoren Mangan, Phosphor und/oder Alkalimetalle in Form ihrer wasserlöslichen Salze (im Normalfall pH-Wert < 7) kann durch Zugabe einer alkalischen Lösung eine Mischung der im Katalysator enthaltenen Hauptbestandteile in Form der Carbonate, Hydroxide oder Oxide ausgefällt werden. Die alkalische Lösung kann durch Lösen von z. B. Alkalicarbonaten oder -hydroxiden, Ammoniak, Ammoniumcarbonat oder Ammoniumhydrogencarbonat oder ähnlichen basischen Salzen in Wasser hergestellt werden. Die Konzentrationen sowohl der Metallsalz - als auch der Fällösung - sollten so eingestellt werden, daß die resultierende Fällmaische noch gerührt werden kann. Werden die Promotoren nicht in diesem Schritt mitgefällt, können sie in einer der weiter unten beschriebenen Verfahrensstufen zugeführt werden. Die Zugabe der basischen Lösung wird so lange fortgesetzt, bis eine vollständige Ausfällung erreicht ist. Das Produkt der Fällung kann erforderlichenfalls nachgerührt, mit üblichen technischen Mitteln abfiltriert und von unerwünschten wasserlöslichen Fremdionen freigewaschen werden.

Der so entstandene Filterkuchen kann dann bei Temperaturen von 50 bis 200°C getrocknet und das so erhaltene Gut vermahlen werden. Alternativ ist eine Aufschlämmung und ein anschließendes Versprühen der Maische in einem Sprühturm möglich. Hierbei entsteht bei Temperaturen zwischen 100 und 600°C ein Sprühpulver. Wird das Versprühen gewählt, so können dem Katalysator auch in diesem Verfahrensschritt die Promotoren Mangan, Phosphor und/ oder Alkalimetalle in Form ihrer Salze beigefügt werden.

Die so erzeugten Pulver können calciniert werden. Die calcinierten Pulver können in verschiedener Weise zu Formkörpern verformt werden. So ist es möglich, die Pulver zu tablettieren, zu extrudieren oder mit Hilfe einer Strangpresse zu Strängen bestimmter Form und Größe zu verpressen. In allen Fällen können dem Pulver Verformungshilfsmittel wie Grafit oder Stearinsäure beigemischt werden.

Die Calcinierung wird in einem Schritt bei Endtemperaturen von 500 bis 1000°C, bevorzugt 800 bis 1000°C, besonders bevorzugt 850 bis 950°C, durchgeführt.

Zur Reduktion werden die calcinierten Katalysatoren bei Raumtemperatur mit Stickstoff gespült und unter Stickstoffatmosphäre ein Druck von 2 bis 10 bar, bevorzugt 4 bis 8 bar, eingestellt.

Anschließend werden in der Regel 2 bis 30 % des Stickstoffstroms, bevorzugt 5 bis 15 %, gegen Wasserstoff ausgetauscht und die Temperatur in der Regel innerhalb von 2 bis 24 h, bevorzugt 5 bis 15 h, von Raumtemperatur auf 80 bis 200°C, bevorzugt 120 bis 160°C, erhöht. Dann wird in der Regel ein weiterer Teil des Stickstoffstroms gegen Wasserstoff ausgetauscht, so daß insgesamt ein Wasserstoffanteil von 30 bis 70 %, bevorzugt 40 bis 60 % erreicht wird. Anschließend wird in der Regel die Temperatur innerhalb von 2 bis 24 h, bevorzugt 5 bis 15 h, auf 200 bis 400°C, bevorzugt 250 bis 350°C erhöht. Diese Endtemperatur wird in der Regel so lange gehalten, bis in dem den Katalysator verlassenden Gasstrom kein Reduktionswasser mehr nachweisbar ist. Anschließend wird in der Regel der Wasserstoffanteil im Gasstrom wieder gegen Stickstoff ausgetauscht und man läßt den reduzierten Katalysator im Stickstoffstrom auf Raumtemperatur abkühlen.

Zur oberflächlichen Anoxidation des reduzierten Katalysators dosiert man dem Stickstoffstrom sukzessive Luft zu, und zwar so langsam, daß die Temperatur im Katalysatorbett den Wert von 60°C nicht überschreitet, also 20 bis 60°C, bevorzugt 20 bis 50°C, besonders bevorzugt 20 bis 40°C. Der Austausch von Stickstoff gegen Luft wird so lange fortgeführt, bis der den Katalysator durchströmende Gasstrom aus 100 % Luft besteht.

Man erhält dadurch Kobaltkatalysatoren mit einer spezifischen Oberfläche von größer oder gleich 12 m²/g, also 12 bis 500 m²/g, bevorzugt 15 bis 200 m²/g, besonders bevorzugt 18 bis 100 m²/g und einer Porosität von größer oder gleich 0,16 cm³/g, also 0,16 bis 1,00 cm³/g, bevorzugt 0,18 bis 0,80 cm³/g, besonders bevorzugt 0,20 bis 0,40 cm³/g.

Die erfindungsgemäßen Katalysatoren zeichnen sich ferner dadurch aus, daß im aktivierten Zustand mindestens 85 Gew.-%, also 85 bis 100 Gew.-%, bevorzugt mindestens 95 Gew.-%, also 95 bis 100 Gew.-% des metallischen Kobalts in hexagonaler Modifikation vorliegt.

Als Formen sind alle geometrischen Körper herstellbar, die sich in Festbettreaktoren einfüllen lassen.

Es eignen sich sowohl Voll- als auch Trägerkatalysatoren für Hydrierungen. Die gilt auch für die Umsetzung von Nitrilen und Iminen mit Wasserstoff zu den entsprechenden Aminen.

Die erfindungsgemäßen Katalysatoren eignen sich als Hydrierkatalysatoren, besonders für Umsetzungen von Nitrilen und/oder Iminen mit Wasserstoff zu primären Aminen bei Temperaturen von 60 bis 150°C und Drücken von 50 bis 300 bar.

Als Kriterium der chemischen Langzeitstabilität (z. B. der Basenstabilität) wurde ein Test entwickelt, der es erlaubt, schon nach kurzer Zeit eine Aussage über das Standzeitverhalten eines Katalysators unter Reaktionsbedingungen zu treffen.

Dieser als Kochtest bezeichnete Kurztest kann folgendermaßen durchgeführt werden:

Ein mit Wasserstoff oberhalb von 200°C reduzierter Katalysator und eine wäßrige Base wie NaOH oder KOH können in einen Autoklaven unter Inertgasatmosphäre eingefüllt und bei ca. 160°C unter autogenem Druck von ca. 5 bar 12 h gehalten werden. Nach Abkühlen, Abdekantieren der Flüssigkeit, Waschen des Katalysators mit Wasser kann die Härte unter Inertgasatmosphäre z. B. unter Stickstoff ermittelt werden.

Ein Katalysator mit einer Schneidhärte von größer oder gleich 30 N, also 30 bis 1000 N, bevorzugt 35 bis 200 N, besonders bevorzugt 40 bis 100 N besitzt erfahrungsgemäß eine ausreichende Langzeitstabilität bei Reaktionen in basischen Medien (Standzeit > 5000 h).

### Beispiele

### Kochtest

In einem 250 ml-Autoklaven mit Tefloneinsatz wurden 10 ml Katalysator in reduzierter Form unter Stickstoff und 100 ml einer 2,5%igen wäßrigen NaOH-Lösung eingefüllt. Die Reduktion des Katalysators wurde zuvor in einer kontinuierlichen Apparatur mit H₂ bei 360°C innerhalb von 5 h durchgeführt. Der verschlossene Autoklav wurde auf 160°C aufgeheizt. Dabei entstand ein autogener Druck von ca. 5 bar. Die Temperatur wurde für 12 h auf 160°C gehalten, nach dem Abkühlen, die Flüssigkeit abdekantiert, der Katalysator mit Wasser gewaschen und anschließend die Härte unter N₂ ermittelt.

### Katalysatorherstellung

Die Angaben der Gewichtsprozente beziehen sich auf die jeweiligen Oxide im geglühten Katalysator, der Phosphor-Gehalt ist als H₃PO₄ angegeben.

### Katalysator A

Durch Auflösen von Kobaltnitrat, Mangannitrat und Phosphorsäure in Wasser wurde eine Lösung hergestellt, die 10 Gew.-% Kobalt, 0,55 Gew.-% Mangan und 0,45 Gew.-% H₃PO₄ enthält. Durch Zugabe einer 20%igen Natriumcarbonatlösung wurde bei einer Termperatur von 50°C gefällt. Der entstandene Niederschlag wurde gewaschen, bis im Waschwasser kein Natrium oder Nitrat mehr nachweisbar war. Der so erhaltene Feststoff wurde mit Wasser angemaischt und in einem Sprühturm versprüht (Eingangstemperatur = 550°C). Das Sprühgut wurde bei 500°C getrocknet, gekollert und im Extruder zu Strängen von 4 mm Durchmesser verformt. Die Stränge wurden bei 100 bis 120°C getrocknet und 1 h bei 900°C calziniert.

Zur Reduktion wurde der calcinierte Katalysator bei Raumtemperatur mit Stickstoff gespült und unter Stickstoffatmossphäre ein Druck von 6 bar eingestellt. Anschließend wurden 10 % des Stickstoffstroms gegen Wasserstoff ausgetauscht und die Temperatur innerhalb von 10 h von Raumtemperatur auf 140°C erhöht. Dann wurden weitere 38 % des Stickstoffstroms gegen Wasserstoff ausgetauscht, so daß insgesamt ein Wasserstoffanteil von 48 % erreicht wurde. Anschließend wurde die Temperatur innerhalb von 10 h von 140°C auf 300°C erhöht. Diese Endtemperatur wurde so lange gehalten, bis in dem den Katalysator verlassenden Gasstrom kein Reduktionswasser mehr nachweisbar war. Anschließend wurde der Wasserstoffanteil im Gasstrom wieder gegen Stickstoff ausgetauscht und der reduzierte Katalysator wurde im Stickstoffstrom auf Raumtemperatur abgekühlt.

Zur oberflächlichen Anoxidation des reduzierten Katalysators wurde dem Stickstoffstrom sukzessive Luft zudosiert, und zwar so langsam, daß die Temperatur im Katalysatorbett den Wert von 60°C nicht überschritt. Der Austausch von Stickstoff gegen Luft wurde so lange fortgeführt, bis der den Katalysator durchströmende Gasstrom aus 100 % Luft bestand.

Der so hergestellte Katalysator enthielt 90,0 Gew.-% Kobalt, 5,4 Gew.-% Mangan, 2,8 % Phosphor und 0,16 % Natrium und besaß eine spezifische Oberfläche von 21,3 m²/g und eine Porosität von 0,22 cm³/g. Im aktivierten Zustand lagen 95 Gew.-% des metallischen Kobalts in der hexagonalen Modifikation vor.

### Katalysator B (Vergleichskatalysator)

Die Herstellung erfolgte analog Katalysator A, jedoch wurde der Katalysator nach der Calzinierung nicht im Wasserstoffstrom reduziert und durch Behandlung im Luftstrom oberflächlich anoxidiert.
Der so hergestellte Katalysator enthielt 90 Gew.-% Kobalt, 5,2 Gew.-% Mangan, 3 % Phosphor und 0,22 % Natrium und besaß eine spezifische Oberfläche von 3,1 m²/g und eine Porosität von 0,13 cm³/g. Im aktivierten Zustand lagen 70 Gew.-% des metallischen Kobalts in der hexagonalen Modifikation vor.

### Katalysator C (Vergleichskatalysator)

Die Herstellung erfolgte analog Katalysator A, jedoch wurde der Katalysator 1 h bei 650°C und 3 h bei 850°C calziniert und danach nicht im Wasserstoffstrom reduziert und durch Behandlung im Luftstrom oberflächlich anoxidiert. Im aktivierten Zustand lagen 80 Gew.-% des metallischen Kobalts in der hexagonalen Modifikation vor.

Der so hergestellte Katalysator enthielt 90,4 Gew.-% Kobalt, 5,1 Gew.-% Mangan, 3,1 % Phosphor und 0,30 % Natrium und besaß eine spezifische Oberfläche von 1,6 m²/g und eine Porosität von 0,11 cm³/g,

### Versuchsdurchführung

Ein vertikaler Rohrreaktor (Durchmesser: 16 mm, Füllhöhe: 50 cm, ölbeheizter Doppelmantel) wurde mit 400 g (200 ml) des Katalysators A gefüllt. Zur Aktivierung des Katalysators wurde drucklos unter Durchleiten von 200 Nl/h Wasserstoff die Temperatur innerhalb von 24 h schrittweise von 100 auf 340°C erhöht und dann 24 h bei 340°C gehalten.

Durch einen vor den Hydrierreaktor geschalteten Rohrreaktor (Durchmesser: 16 mm, Füllhöhe: 100 cm, ölbeheizter Doppelmantel), der mit 37 g (50 ml) Titandioxid in Form von 1,5 mm-Strängen gefüllt war, wurden bei einem Druck von 250 bar und einer Temperatur von 80°C stündlich von unten nach oben 80 g Isophoron- nitril (Reinheit 99,0 %) und 270 g flüssiges Ammoniak gepumpt (Katalysatorbelastung 0,4 kg/l x h). Anschließend wurden stündlich 100 Nl/h (4,5 mol) Wasserstoff zugefahren und der Austrag aus dem vorgeschalteten Iminierungsreaktor bei einem Druck von 250 bar und einer Temperatur von 130°C von unten nach oben durch den Hydrierreaktor gefahren. Nach Entspannen auf Normaldruck wurde der Ammoniak abdestilliert und der Hydrieraustrag gaschromatographisch analysiert.

Die Versuche mit den Vergleichskatalysatoren B und C wurden analog durchgeführt.

Katalysatoren mit ausreichender Hydrieraktivität zeichnen sich dadurch aus, daß der Gehalt eines als Aminonitril bezeichneten Zwischenproduktes weniger als 500 ppm beträgt.

Die Ergebnisse sind in der nachfolgenden Tabelle zusammengestellt:

| Katalysator | Schneidhärte nach Kochtest [N] | Oberfläche [m²/g] | Porosität [cm³/g] | Aminonitril [ppm] | Ausbeute an Isophorondiamin [%] | hexagonales Kobalt (im aktivierten Zustand) [%] |
|---|---|---|---|---|---|---|
| A | 49 | 21,3 | 0,22 | < 100 | 99 | 95 |
| B | 1 | 3,1 | 0,13 | 10000 | 95 | 70 |
| C | 20 | 1,6 | 0,11 | 200 | 99 | 80 |

## Patentansprüche

1. Kobaltkatalysatoren, deren katalytisch aktive Masse aus 55 bis 98 Gew.-% Kobalt, 0,2 bis 15 Gew.-% Phosphor, 0,2 bis 15 Gew.-% Mangan und 0,05 bis 5 Gew.-% Alkali, berechnet als Oxid, besteht, **dadurch gekennzeichnet, daß** man die kalzinierten Katalysatoren bei Endtemperaturen von 200 bis 400°C im Wasserstoffstrom reduziert und anschließend durch Behandlung im Luftstrom bei Endtemperaturen von 20 bis 60°C oberflächlich anoxidiert, daß die Kobaltkatalysatoren eine spezifische Oberfläche von größer oder gleich 12 m²/g besitzen und daß die Kobaltkatalysatoren nach Durchführung eines Kochtests zur Bestimmung der Basenstabilität eine Schneidhärte von größer oder gleich 30 N aufweisen.

2. Kobaltkatalysatoren nach Anspruch 1, **dadurch gekennzeichnet, daß** deren katalytisch aktive Masse aus 75 bis 95 Gew.-% Kobalt, 0,5 bis 10 Gew.-% Phosphor, 2 bis 10 Gew.-% Mangan und 0,1 bis 3 Gew.-% Alkali, berechnet als Oxid, besteht.

3. Kobaltkatalysatoren nach Anspruch 1, **dadurch gekennzeichnet, daß** deren katalytisch aktive Masse aus 85 bis 95 Gew.-% Kobalt, 1 bis 6 Gew.-% Phosphor, 3 bis 8 Gew.-% Mangan und 0,13 bis 1 Gew.-% Alkali, berechnet als Oxid, besteht.

4. Kobaltkatalysatoren nach Anspruch 1, **dadurch gekennzeichnet, daß** man als Alkali Lithium, Natrium, Kalium und/oder Cäsium einsetzt.

5. Kobaltkatalysatoren nach Anspruch 1, **dadurch gekennzeichnet, daß** die Kobaltkatalysatoren eine Porosität von größer oder gleich 0,16 cm³/g aufweisen.

6. Kobaltkatalysatoren nach Anspruch 1, **dadurch gekennzeichnet, daß** mindestens 85 Gew.-% des metallischen Kobalts im aktivierten Zustand in hexagonaler Modifikation vorliegt.

7. Kobaltkatalysatoren nach Anspruch 1, **dadurch gekennzeichnet, daß** mindestens 95 Gew.-% des metallischen Kobalts im aktivierten Zustand in hexagonaler Modifikation vorliegt.

## Claims

1. A cobalt catalyst whose catalytically active composition comprises from 55 to 98 % by weight of cobalt, from 0.2 to 15 % by weight of phosphorus, from 0.2 to 15 % by weight of manganese and from 0.05 to 5 % by weight of alkali metal, calculated as oxide, wherein the calcined catalysts are reduced in a stream of hydrogen at a final temperature of from 200 to 400°C and are subsequently surface-oxidized by treatment in a stream of air at final temperatures of from 20 to 60°C, wherein the cobalt catalyst has a specific surface area of greater than or equal to 12 m²/g and wherein the cobalt catalyst has a cutting hardness of greater than or equal to 30 N after carrying out an autoclave test for determining the base stability.

2. A cobalt catalyst as claimed in claim 1, whose catalytically active composition comprises from 75 to 95 % by weight of cobalt, from 0.5 to 10 % by weight of phosphorus, from 2 to 10 % by weight of manganese and from 0.1 to 3 % by weight of alkali metal, calculated as oxide.

3. A cobalt catalyst as claimed in claim 1, whose catalytically active composition comprises from 85 to 95 % by weight of cobalt, from 1 to 6 % by weight of phosphorus, from 3 to 8 % by weight of manganese and from 0.13 to 1 % by weight of alkali metal, calculated as oxide.

4. A cobalt catalyst as claimed in claim 1, wherein the alkali metal used is lithium, sodium, potassium and/or cesium.

5. A cobalt catalyst as claimed in claim 1, which has a porosity of greater than or equal to 0.16 cm³/g.

6. A cobalt catalyst as claimed in claim 1, wherein at least 85 % by weight of the metallic cobalt in the activated state is in the hexagonal modification.

7. A cobalt catalyst as claimed in claim 1, wherein at least 95 % by weight of the metallic cobalt in the activated state is in the hexagonal modification.

## Revendications

1. Catalyseurs au cobalt, dont la masse catalytiquement active est constituée de 55 à 98 % en poids de cobalt, de 0,2 à 15 % en poids de phosphore, de 0,2 à 15 % en poids de manganèse et de 0,05 à 5 % en poids d'un métal alcalin, calculés en oxyde, **caractérisés en ce qu'**on réduit les catalyseurs calcinés dans un courant d'hydrogène à des températures finales de 200 à 400°C, puis, par traitement dans un courant d'air, on procède à une oxydation anodique en surface à des températures finales de 20 à 60°C ; que les catalyseurs au cobalt ont une aire spécifique supérieure ou égale à 12 m²/g ; et que les catalyseurs au cobalt, après mise en oeuvre d'un essai à ébullition pour déterminer la stabilité vis-à-vis des bases, présentent une dureté à la tranche supérieure ou égale à 30 N.

2. Catalyseurs au cobalt selon la revendication 1, **caractérisés en ce que** leur masse catalytiquement active est constituée de 75 à 95 % en poids de cobalt, de 0,5 à 10 % en poids de phosphore, de 2 à 10 % en poids de manganèse et de 0,1 à 3 % en poids d'un métal alcalin, calculés en oxyde.

3. Catalyseurs au cobalt selon la revendication 1, **caractérisés en ce que** leur masse catalytiquement active est constituée de 85 à 95 % en poids de cobalt, de 1 à 6 % en poids de phosphore, de 3 à 8 % en poids de manganèse et de 0,13 à 1 % en poids d'un métal alcalin, calculés en oxyde.

4. Catalyseurs au cobalt selon la revendication 1, **caractérisés en ce qu'**on utilise en tant que métal alcalin le lithium, le sodium, le potassium et/ou le césium.

5. Catalyseurs au cobalt selon la revendication 1, **caractérisés en ce que** les catalyseurs au cobalt ont une porosité supérieure ou égale à 0,16 cm³/g.

6. Catalyseurs au cobalt selon la revendication 1, **caractérisés en ce qu'**au moins 85 % en poids du cobalt métallique se présentent à l'état activé sous la forme hexagonale.

7. Catalyseurs au cobalt selon la revendication 1, **caractérisés en ce qu'**au moins 95 % en poids du cobalt métallique se présentent à l'état activé sous la forme hexagonale.
